# EUROPEAN PATENT SPECIFICATION

(11) **EP 2 555 770 B1**
(45) Date of publication and mention of the grant of the patent: **01.11.2017**
(21) Application number: 11715353.6
(22) Date of filing: 06.04.2011
(51) Int. Cl.: A61K 31/407, A61P 27/02, A61K 9/00, A61K 9/08, A61K 47/38

(54) **KETOROLAC COMPOSITIONS FOR CORNEAL WOUND HEALING**
KETOROLAC-ZUSAMMENSETZUNGEN ZUR ABHEILUNG VON HORNHAUTWUNDEN
COMPOSITIONS DE KÉTOROLAC POUR LA CICATRISATION DE LÉSIONS CORNÉENNES

(30) Priority: 11.06.2010 US 353723 P; 30.04.2010 US 329992 P; 09.04.2010 US 322628 P
(43) Date of publication of application: 13.02.2013
(73) Proprietor: ALLERGAN, INC., Irvine, CA 92612 (US)
(72) Inventor: GRAHAM, Richard, S., Irvine California 92612 (US); HOLLANDER, David, Tustin California 92782 (US); VILLANUEVA, Linda, Huntington Beach California 92649 (US); FARNES, Eldon, Quinn, Laguna Beach California 92651 (US); ATTAR, Mayssa, Placentia California 92870 (US); SCHIFFMAN, Rhett, M., Laguna Beach California 92651 (US); CHANG, Chin-Ming, Tustin California 92782 (US); WELTY, Devin, F., Foothill Ranch California 92610 (US)
(74) Representative: Hoffmann Eitle
(86) International application number: PCT/US2011/031453
(87) International publication number: WO 2011/127196

(56) References cited:
- WO-A2-2009/111418
- AU-B1- 2009 202 969
- XU K ET AL: "Ex vivo corneal epithelial wound healing following exposure to ophthalmic nonsteroidal anti-inflammatory drugs", CLINICAL OPHTHALMOLOGY 2011 DOVE MEDICAL PRESS NZL LNKD- DOI:10.2147/OPTH.S16778, vol. 5, no. 1, 1 May 2011 (2011-05-01), pages 269-274, XP002638530, ISSN: 1177-5467
- GARRETT Q ET AL: "Carboxymethyl cellulose stimulates rabbit corneal epithelial wound healing", CURRENT EYE RESEARCH 200807 GB LNKD- DOI:10.1080/02713680802140213, vol. 33, no. 7, July 2008 (2008-07), pages 567-573, XP009148776, ISSN: 0271-3683
- DONNENFELD E D ET AL: "Double-masked study of the effects of nepafenac 0.1% and ketorolac 0.4% on corneal epithelial wound healing and pain after photorefractive keratectomy", ADVANCES IN THERAPY, HEALTH COMMUNICATIONS, METUCHEN, NJ, US, vol. 24, no. 4, 1 July 2007 (2007-07-01), pages 852-862, XP009148763, ISSN: 0741-238X, DOI: DOI:10.1007/BF02849978
- LEE W B ET AL: "Corneal ulceration and perforation with ketorolac tromethamine [4]", CORNEA 200612 US LNKD- DOI:10.1097/01.ICO.0000229983.30957.A2, vol. 25, no. 10, December 2006 (2006-12), page 1268, XP009148759, ISSN: 0277-3740
- CHA S -H ET AL: "Corneal epithelial cellular dysfunction from benzalkonium chloride (BAC) in vitro", CLINICAL AND EXPERIMENTAL OPHTHALMOLOGY 200404 AU LNKD- DOI:10.1111/J.1442-9071.2004.00782.X, vol. 32, no. 2, April 2004 (2004-04), pages 180-184, XP002638531, ISSN: 1442-6404
- KUSANO M ET AL: "Evaluation of acute corneal barrier change induced by topically applied preservatives using corneal transepithelial electric resistance in vivo", CORNEA 2010 LIPPINCOTT WILLIAMS AND WILKINS USA LNKD- DOI:10.1097/ICO.0B013E3181A3C3E6, vol. 29, no. 1, January 2010 (2010-01), pages 80-85, XP009148774, ISSN: 0277-3740
- "Acuvail (keterolac tromethamine)", , 17 September 2010 (2010-09-17), XP055085192, Internet Wayback machine Retrieved from the Internet: URL:http://web.archive.org/web/20100917233 536/http://www.medilexicon.com/drugs/acuva il.php [retrieved on 2013-10-24]
- Anonymous: "Acuvail (ketorolac tromethamine) - Drug information from MediLexicon", , 17 September 2010 (2010-09-17), XP055153928, Retrieved from the Internet: URL:https://web.archive.org/web/2010091723 3536/http://www.medilexicon.com/drugs/acuv ail.php [retrieved on 2014-11-19]

## Description

### Field of the Invention

This invention relates to pharmaceutical compositions for use in a method of promoting corneal wound healing in a patient. More particularly, this invention relates to topical ophthalmic solutions according to the claims comprising 5-benzoyl-2,3-dihydro-1*H-*pyrrolizine-1-carboxylic acid tromethamine, otherwise known as ketorolac tromethamine, for use in a method of promoting corneal wound healing and corneal repair in a patient.

### Background of the Related Art

Nonsteroidal anti-inflammatory drugs (NSAIDS) have anti-inflammatory, analgesic, and antipyretic properties. NSAIDS primarily act as cycloxygenase (COX) inhibitors and limit the production of endogenous prostaglandins (PGs) in the arachidonic acid cascade. Topical NSAIDS are commonly used in ophthalmology to control postoperative inflammation, reduce pain following refractive surgery, inhibit intraoperative miosis, and treat allergic conjunctivitis. Commercially available topical NSAIDS may differ in the type and concentration of the active agent, concentration of preservative, pH, and/or additional surfactants.

Topical NSAIDS have been associated with corneal complications which include superficial punctate keratopathy, subepithelial infiltrates, epithelial defects, delayed epithelial healing, corneal anesthesia, ulceration and perforation. While corneal epithelial toxicity has been attributed to both the active agents and the preservatives within topical formulations as well as preexisting systemic conditions, keratitis, corneal ulceration and perforation have all been reported in patients using preservative-free formulations. Animal studies have suggested that the corneal toxicity of topical NSAIDS may result from an upregulation of matrix metalloproteinases (MMPs), and endopeptidases involved in the remodeling of the corneal extracellular matrix.

As topical NSAIDs are commonly used in the perioperative setting, it is critical to develop formulations that do not inhibit epithelial healing but in fact enhance epithelial wound healing. Topical ketorolac, first introduced in 1993, remains the most commonly used ophthalmic NSAID. Ophthalmic ketorolac 0.45% solution (Acuvail®, Allergan, Inc; Irvine, CA) was developed to maintain the efficacy of prior formulations of ketorolac while enhancing tolerability and reducing the dosing regimen. Changes in the new formulation, ketorolac 0.45%, include the removal of the preservative (BAK) and the surfactant (octoxynal 40) and the addition of carboxymethyl cellulose (CMC) which prolongs drug retention on the eye, protects the ocular surface, and may also promote corneal re-epithelialization

In Advances in Therapy, 2007, Jul-Aug, 24(4):852-62, Donnenfeld et al discussed a double-masked study of the effects of nepafenac 0.1% and ketorolac 0.4% on corneal epithelial wound healing and pain after photorefractive keratectomy. In this study, nepafenac 0.1% and ketorolac tromethamine 0.4% were compared in terms of their effects on corneal reepithelialization and pain after photorefractive keratectomy (PRK) in a randomized, double-masked, contralateral eye, multicenter study. A total of 40 healthy adult patients who were undergoing sequential bilateral PRK received nepafenac 0.1% and ketorolac 0.4% in contralateral eyes, 1 drop 3 times daily for 3 d after bandage contact lens insertion. Patients were assessed on postoperative days 1, 3, 4, 5, and 7. At each visit, patients provided a general rating of pain. Each patient also assessed the sensation of each eyedrop following instillation (after-drop pain, irritation, burning/stinging, and overall comfort). Starting on day 3, epithelial defect size was assessed. Mean epithelial defect size was similar between treatments at each postoperative visit (P>.05). The average time-to-healing was 4.18 d for nepafenac 0.1 % and 4.00 d for ketorolac 0.4% (P=.3134). No statistical difference was observed between nepafenac 0.1% and ketorolac 0.4% in mean postoperative pain scores (P>.05). On day 3, the nepafenac 0.1% group had significantly lower mean sensation scores than did the ketorolac 0.4% group for after-drop pain (P=.0090), irritation (P=.0007), and burning/ stinging (P=.0003). Mean overall comfort score was also significantly better for nepafenac 0.1% on day 3 (7.43 vs 6.41; P<.0001). The authors concluded that nepafenac 0.1% and ketorolac 0.4% provide postoperative pain relief after PRK surgery without associated adverse effects on corneal epithelial healing, and indicated that nepafenac 0.1% treatment may offer greater comfort upon instillation in patients who have undergone PRK.

### Summary of the Invention

The present invention provides an aqueous ophthalmic formulation according to the claims for use in a method of promoting corneal wound healing in a patient comprising an effective amount of ketorolac tromethamine but having an optimized concentration of keterolac in comparison other commercially available keterolac products. The aqueous ophthalmic solution for use in the present invention comprises carboxymethyl cellulose, e.g. sodium carboxymethyl cellulose, having a pH within the range of from 6.8 to 7.4, which is comfortable when topically applied to the eye of a patient, wherein the concentration of carboxymethyl cellulose and, preferably, the pH, is selected to provide an increased absorption of ketorolac in the eye of a patient as compared to a comparative keterolac solution that differs in whole or in part in not including the carboxymethyl cellulose. That is, the absorption of keterolac may be 130% or greater than the absorption of a comparative aqueous ketorolac ophthalmic solution having the same or higher concentration of ketorolac. The aqueous ophthalmic solution for use in the present invention contains no preservative.

More preferably, the aqueous ophthalmic solution for use in this invention has a pH within the range of from 6.8 to 7.4, particularly 6.8.

More preferably, the aqueous ophthalmic solution for use in the present invention has a concentration of carboxymethyl cellulose of from 0.1 to 2 percent, by weight, even more preferably from 0.5 to 1.5 percent, by weight, and most preferably 0.5% w/v.

Even more preferably, the aqueous ophthalmic solution for use in the present invention comprises a mixture of medium viscosity and high viscosity sodium carboxymethyl cellulose.

The aqueous ophthalmic solution for use in the invention comprises an effective amount of ketorolac of 0.45 percent, by weight.

More preferably, the aqueous ophthalmic solution for use in the invention has a viscosity of from 5 to 50 cps, preferably from 10 to 30 cps.

It has been surprisingly discovered that optimizing the concentration of ketorolac tromethamine reduces the occurrence of adverse events while maintaining clinical efficacy. Additionally, it has been discovered that the optimized concentration of ketorolac tromethamine in combination with carboxymethyl cellulose offers surprising and clear benefits in terms of formulation in that no preservative, chelating agent, and surfactant are required for formulation. Thus, finding a way to increase the absorption of ketorolac benefits the patient who can use a solution having an optimized concentration of ketorolac and obtain similar results in terms of efficiency as compared to a ketorolac solution having a higher concentration of ketorolac.

Thus, this invention relates to an aqueous topical ophthalmic composition for use in a method of promoting corneal wound healing in a patient comprising 0.45 % ketorolac tromethamine by weight/volume. The composition for use in the present invention also contains from 0.2 to 2 percent by weight/volume, more preferably from 0.5 to 1.5 percent by weight/volume and most preferably 0.5 % w/v percent of mixtures of medium and high molecular weight sodium carboxymethyl cellulose. Another aspect of this invention relates to a composition for use in a method of treating or preventing ocular pain in a person while promoting epithelial wound healing before and/or following surgery or a corneal wound comprising topically administering (qd, bid, tid, qid) to a patient a sterile composition comprising 0.45% w/v ketorolac tromethamine in combination with from 0.2 to 2 percent, by weight/volume, preferably from 0.5 to 1.5 percent by weight/volume, and most preferably 0.5% percent by weight/volume, sodium carboxymethyl cellulose and mixtures thereof.

Of particular interest in relationship to this invention is the aqueous topical ophthalmic compositions of 0.45% (w/v) ketorolac tromethamine for use in the treatment of ocular pain in postoperative photorefractive keratectomy (PRK) surgery patients and cataract surgery patients which improves corneal wound healing. It is surprising that the lower concentration of ketorolac as compared to the Acular® product, discussed herein, would reduce the incidence of adverse events and enhance comfort while maintaining clinical efficacy. Two drops (0.1 mL) of 0.5% ketorolac tromethamine ophthalmic solution instilled into the eyes of patients 12 hours and 1 hour prior to cataract extraction achieved measurable levels in 8 of 9 patients' eyes (mean ketorolac concentration 95 ng/mL aqueous humor, range 40 to 170 ng/mL). Ocular administration of ketorolac tromethamine reduces prostaglandin E₂ (PGE₂) levels in aqueous humor. The mean concentration of PGE₂ was 80 pg/mL in the aqueous humor of eyes receiving vehicle and 28 pg/mL in the eyes receiving 0.5% ketorolac tromethamine ophthalmic solution.

Ocular administration of 0.45% w/v ketorolac tromethamine ophthalmic solution increases relative bioavailability of ketorolac in the aqueous humor of rabbits to greater than 200% and in the iris-ciliary body to nearly 300%, compared with 0.5% ketorolac tromethamine ophthalmic solution. This enhanced ketorolac bioavailability allows for a reduction in dosing frequency from QID with 0.5% ketorolac tromethamine ophthalmic solution to BID with 0.45% keterolac solution. Preclinical data indicate systemic ketorolac exposure levels achieved following ocular administration of 0.45% keterolac solution are comparable to levels achieved with 0.5% ketorolac tromethamine ophthalmic solution.

Some embodiments of the invention are as follows:
1. A composition for use in promoting corneal wound healing in a patient comprising an aqueous solution of 0.45 % w/v ketorolac tromethamine, wherein the composition further comprising carboxymethyl cellulose and wherein the composition contains no preservative.
2. The topical aqueous ophthalmic solution for use of paragraph 1 wherein the carboxymethyl cellulose is a combination of medium and high viscosity carboxymethyl cellulose.
3. The topical aqueous ophthalmic solution for use of paragraph 2 having a pH between 6.8 and 7.4.
4. The topical aqueous ophthalmic solution for use of paragraphs 2 and 3 wherein the concentration of carboxymethylcellulose is from 0.2 to 2 percent by weight.
5. The topical aqueous ophthalmic solution for use of paragraph 3 having a pH of approximately 6.8.
6. The topical aqueous solution for use of paragraphs 1 to 5 wherein the solution is surfactant, preservative and chelator free.
7. The topical aqueous solution for use of paragraphs 2, 5 and 6 further comprising a mixture of medium and high viscosity sodium carboxymethyl cellulose, sodium chloride, sodium citrate dehydrate, sodium hydroxide, hydrochloric acid and purified water.
8) The topical aqueous solution for use of paragraph 7 wherein the combination of carboxymethyl cellulose and keterolac increases the absorption of ketorolac in the eye of a patient more than a solution of keterolac alone without carboxymethyl cellulose.
9) The topical aqueous solution for use of paragraphs 1 to 3 and 6 to 8 wherein the keterolac tromethamine is present as a racemic mixture of R-(+) and S-(-)- ketorolac tromethamine.
10) The topical aqueous solution for use of paragraphs 1 to 3 and 6 to 9 wherein the keterolac tromethamine is present in a mixture of crystal forms.
11) The topical aqueous solution for use of paragraph 2 wherein the viscosity is from 10 to 30 cps.
12) The topical aqueous solution for use of paragraphs 1, 2, 4 and 6 wherein the carboxymethylcellulose is present in the amount of 0.5% percent by weight.
13) The topical aqueous solution for use of paragraphs 2 and 7 wherein the solution may be administered at least once a day before or after eye surgery to promote healing of epithelial cell growth of the cornea.
14) The topical aqueous solution for use of paragraph 1, 2, 7, 8 and 11 wherein administration of the solution to the eye accelerates corneal wound healing of the eye after surgery to a greater degree than keterolac solutions containing no carboxymethyl cellulose.
15) A composition for use in a method of accelerating the healing of corneal epithelial tissues in a patient after eye surgery by administering the composition, which comprises 0.45% w/v ketorolac tromethamine and carboxymethyl cellulose and no preservative wherein the healing of corneal epithelial tissues in the patient progresses more rapidly than it would without administration of a ketorolac and carboxymethyl cellulose composition.
16) The composition for use according to paragraph 15 wherein the composition is a topical aqueous solution and wherein the topical aqueous solution is instilled at least twice daily to the patient's eyes.
17) The composition for use according to paragraph 15 wherein the carboxymethyl cellulose is a combination of medium and high viscosity carboxymethyl cellulose.
18) The composition for use according to paragraph 15 wherein the eye surgery is selected from the group consisting of laser eye surgery, cataract surgery, glaucoma surgery, refractive surgery, corneal surgery, vitreoretinal surgery, eye muscle surgery, oculoplastic surgery, surgery involving the lacrimal apparatus, and photorefractive surgery.
19) The composition for use according to paragraph 15 further comprising a mixture of medium and high viscosity sodium carboxymethyl cellulose, sodium chloride, sodium citrate dehydrate, sodium hydroxide, hydrochloric acid and purified water.

### Brief Description of the Drawings

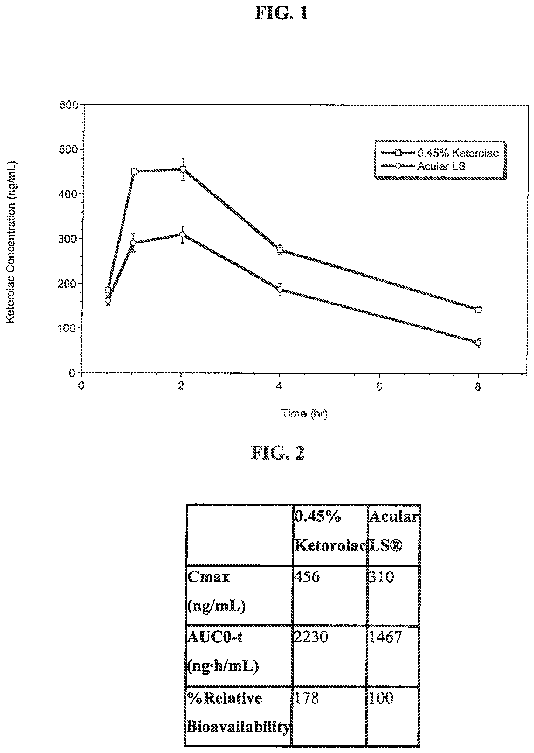

Figure 1 shows the ocular pharmacokinetics of the results in Example 7 of the increased and prolonged keterolac exposure in the aqueous humor of the 0.45% w/v keterolac solution in comparison to ACULAR LS®;
Figure 2 shows the results of Fig. 1 in table form of Cmax, AUC and percent relative bioavailability of the ocular pharmacokinetics in Example 7 of the aqueous humor relative bioavailability of 0.45% w/v keterolac solution in comparison to ACULAR LS®;

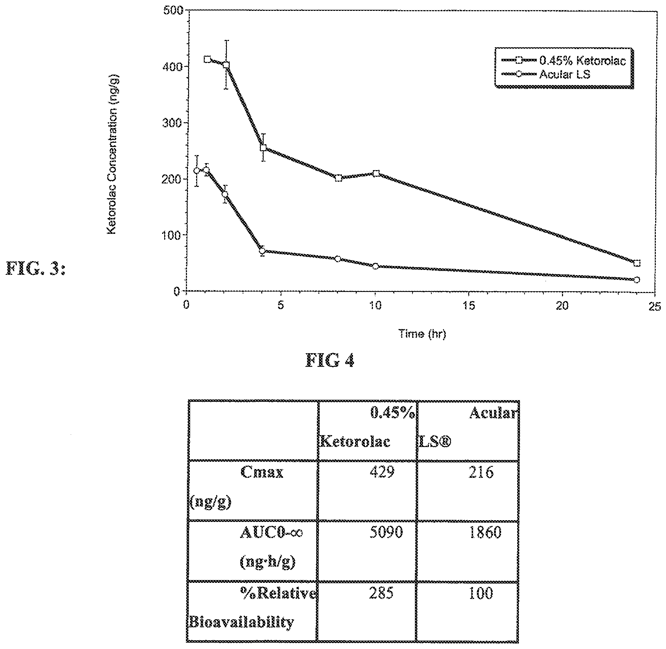

Figure 3 shows the ocular pharmacokinetics of the results in Example 7 of the increased and prolonged keterolac exposure in the iris-ciliary body of 0.45% w/v keterolac solution in comparison to ACULAR LS®;
Figure 4 shows the results of Fig. 3 in table form of Cmax, AUC and percent relative bioavailability of the increased and prolonged exposure in the iris ciliary body of 0.45% w/v keterolac solution in comparison to ACULAR LS®;

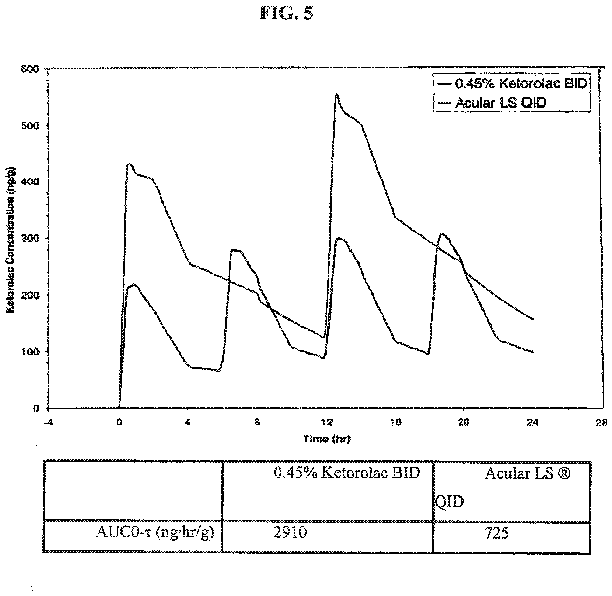

Figure 5 shows a multiple dose simulation of Example 7 of 0.45% keterolac BID in comparison to ACULAR LS® QID in the iris ciliary body;

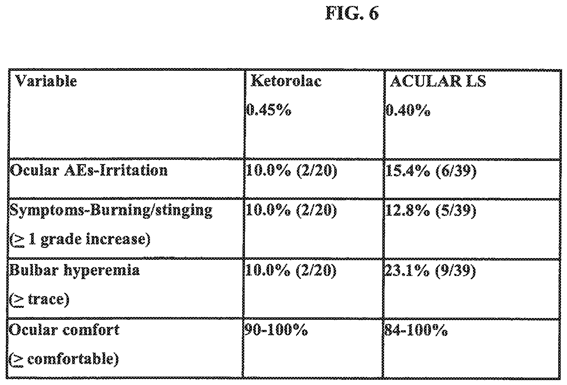

Figure 6 shows safety and tolerability results in human clinical trials of 0.45% w/v keterolac solution vs. ACULAR LS;
Figure 7 shows representative images of corneas with original wound and those following treatment with Triton X-100 1%;
Figure 8 shows representative images of corneas treated with ketorolac 0.5%, 0.4%, and 0.45% (a) and corresponding intensity of the wound area staining (b) are shown; and
Figure 9 shows remaining epithelial wound area in corneas treated with nepafenac 0.1%, Bromfenac 0.09%, and Ketorolac 0.45%.

### Detailed Description of the Invention

During the reformulation of Allergan's marketed Acular LS® product (0.40 % w/v keterolac) it was surprisingly found that a test formulation containing 0.45% ketorolac tromethamine and sodium carboxymethylcellulose (NaCMC) exhibited significantly better ocular absorption in rabbits than did the currently marketed product, i.e. Acular LS®. It has also been surprisingly discovered that certain formulations of ketorolac aid in corneal epithelial wound healing.

Since the viscosities of the two test solutions were virtually identical, the mechanism for achieving increased ocular penetration compared to the control formulation cannot be accounted for only by the viscosity of the test solutions. In fact, a comparison of two identical carboxymethyl cellulose-containing solutions which differ only in having viscosity of 11 and 22 cps shows similar absorption of ketorolac into the aqueous humor. While not wishing to be bound by theory, it is believed that there is a functional relationship between the sodium carboxymethyl cellulose and either the ketorolac or some component of the ocular surface that facilitates absorption of ketorolac.

All of the aqueous topical ophthalmic solutions of this disclosure are contemplated for use in treating or preventing ocular pain. Preferably, all of the solutions of this disclosure are contemplated for use when said ocular pain is a result of photorefractive keratectomy surgery (PRK) or cataract surgery. The aqueous ophthalmic solution is for use in a method of promoting corneal wound healing in a patient according to the present invention.

One important aspect of this disclosure is that the solutions of the present disclosure have a concentration of ketorolac tromethamine which is optimized to reduce side effects, while maintaining clinical efficacy in treating ocular pain. As such, the concentration of ketorolac tromethamine in compositions for use in this invention is 0.45% ketorolac tromethamine, by weight/volume.

Carboxymethyl cellulose (CMC) is a carboxymethyl derivative of cellulose formed by the reaction of cellulose with alkali and chloroacetic acid. As a result of said reaction, carboxymethyl groups are bound to some of the hydroxyl groups of the glucopyranose units that make up the backbone of cellulose. The degree of substitution of carboxymethyl varies from 0.6 to 0.95 per glucopyranose unit. CMC is used in aqueous solutions usually as the sodium salt to increase viscosity.

Carboxymethyl cellulose is available in various molecular weights. Low molecular weight carboxymethyl cellulose has an average Mw of 90,000 and a 2% solution thereof will have a viscosity of 1.1 cP at 25° C. Medium weight carboxymethyl cellulose has an average Mw of 250,000. High molecular weight carboxymethylcellulose has an average Mw of 700,000 and a 2% solution will have a viscosity of 12 cP at 25° C.

For the purpose of the present invention, it is desirable to use a mixture of medium and high molecular weight sodium carboxymethyl cellulose. For example, from 25/75 to 75/25 carboxymethyl cellulose, preferably from 30/70 to 70/30 and most preferably 35/65 medium/high molecular weight sodium carboxymethyl cellulose.

The fact that the concentration of ketorolac tromethamine in compositions for use in this invention achieves greater or equal absorption of ketorolac into the aqueous humor of the eye and includes carboxymethyl cellulose, allows the solutions for use in the present invention to be prepared with no preservative, surfactant and chelating agent. This is a significant advantage over prior art keterolac formulations as preservatives, surfactants and chelating agents can cause irritation to the eye resulting in less patient compliance and less effectiveness of prior art keterolac formulations.

The term preservative has the meaning commonly understood in the ophthalmic art. Preservatives are used to prevent bacterial contamination in multiple-use ophthalmic preparations, and, while not intending to be limiting, examples include benzalkonium chloride, stabilized oxychloro complexes (otherwise known as Purite®), phenylmercuric acetate, chlorobutanol, benzyl alcohol, parabens, and thimerosal. The keterolac solution for use in the present invention is preservative free.

The term surfactant used in this invention has the meaning commonly understood in the art. Surfactants are used to help solubilize the therapeutically active agent or other insoluble components of the composition. Anionic, cationic, amphoteric, zwitterionic, and nonionic surfactants may all be used in this invention. If a surfactant is included in the solutions for use in the invention, preferably, a nonionic surfactant is used. Some examples of useful nonionic surfactants are polysorbates, poloxamers, alcohol ethoxylates, ethylene glycol-propylene glycol block copolymers, fatty acid amides, and alkylphenol ethoxylates, and phospholipids. Most preferably, the surfactant is an octylphenol ethoxylate with an average of 40 ethoxylate groups. This type of surfactant, also known as octoxynol-40 or Igepal CA-897®, can be purchased under the Igepal CA-897® tradename from Rhône-Poulenc. Preferably, the keterolac solution for use in the present invention is surfactant free.

The term chelating agent refers to a compound that is capable of complexing a metal, as understood by those of ordinary skill in the chemical art. Chelating agents are used in ophthalmic compositions to enhance preservative effectiveness. While not intending to be limiting, some useful chelating agents for the purposes of this invention are edetate salts like edetate disodium, edetate calcium disodium, edetate sodium, edetate trisodium, and edetate dipotassium. Preferably, the keterolac solution for use in the present invention is chelator free.

In addition to surfactants, preservatives, and chelating agents, tonicity agents and other excipients are often used in ophthalmic compositions. Tonicity agents are often used in ophthalmic compositions to adjust the concentration of dissolved material to the desired isotonic range. Tonicity agents are known to those skilled in the ophthalmic art, and, while not intending to be limiting, some examples include glycerin, mannitol, sorbitol, sodium chloride, and other electrolytes. Preferably, the tonicity agent is sodium chloride.

The most preferred embodiment of this invention relates to an aqueous topical ophthalmic composition for use in a method of promoting corneal wound healing in a patient consisting of 0.45% (w/v) of ketorolac tromethamine, 0.5 % w/v of carboxymethylcellulose sodium, e.g. a mixture of medium and high viscosity sodium carboxymethyl cellulose, sodium chloride, sodium citrate dehydrate, sodium hydroxide, hydrochloric acid and purified water.

### Example 1

Example 1 does not form part of the invention and is provided for information purposes only. Unless otherwise specified, all steps in this procedure were carried out at room temperature. The following procedure was followed in accordance with the amounts listed in Table 1 below. Purified water was charged into the main batch vessel. Mixing was initiated to produce a vortex sufficient to disperse and/or dissolve all product ingredients without excessive aeration or foam formation. The following components were added directly into the vortex in order, allowing each to dissolve before adding the next: sodium chloride, calcium chloride, dihydrate magnesium chloride, hexahydrate, boric acid, sodium borate, sodium carboxymethyl cellulose as a an percent aqueous solution comprising including a mixture of 65% medium molecular weight and 35% high molecular weight carboxymethyl cellulose. The solution was mixed for no longer than 15 minutes. A specified amount of 1N sodium hydroxide, was then added. The pH was checked and, if needed, was adjusted to 7.3 with 1N sodium hydroxide or 1N hydrochloric acid. Ketorolac tromethamine was then added based on "as is" assay and mixed until completely dissolved based on visual inspection. When dissolved, the solution pH was again checked and if needed adjusted to pH 7.3 - 7.5 (final target pH is 7.4) with 1N sodium hydroxide or 1N hydrochloric acid. Purified water was then added to bring the bulk solution to final volume and allowed to mix for at least 15 minutes to ensure uniformity. The solution was then sterile filtered for use.

**Table 1. 0.4% Ketorolac Tromethamine Ophthalmic Solution**

| | |
|---|---|
| Keterolac Tromethamine | 0.4% |
| CMC, Med Visc. | .65% |
| CMC Low Visc. | .35% |
| Potassium chloride | 0.14% |
| Calcium chloride, dihydrate | 0.060% |
| Magnesium chloride, hexahydrate | 0.060% |
| Boric acid | .060% |
| Sodium borate | .1225% |

### Example 2

Example 2 does not form part of the invention and is provided for information purposes only. Unless otherwise specified, all steps in this procedure were carried out at room temperature. The following procedure was followed in accordance with the amounts listed in Table 2 below. Purified water at 90% of batch size was charged into the main batch vessel. Mixing was initiated to produce a vortex sufficient to disperse and/or dissolve all product ingredients without excessive aeration or foam formation. The following components were added directly into the vortex in order, allowing each to dissolve before adding the next: sodium chloride, edetate disodium, octoxynol-40 (as a 70% stock solution) and benzalkonium chloride (as a 10 % stock solution). The amount of benzalkonium chloride added took into account the assay of the stock solution used. The solution was mixed for no longer than 15 minutes. A specified amount of 1N sodium hydroxide, 1.85 mL per liter of final bulk product, was then added. The pH was checked and if needed was adjusted to 10.7 - 11.0 with 1N sodium hydroxide or 1N hydrochloric acid. Ketorolac tromethamine was then added based on "as is" assay and mixed until completely dissolved based on visual inspection. When dissolved, the solution pH was again checked and if needed adjusted to pH 7.3 - 7.5 (final target pH is 7.4) with 1N sodium hydroxide or 1N hydrochloric acid. Purified water was then added to bring the bulk solution to final volume and allowed to mix for at least 15 minutes to ensure uniformity. The solution was then sterile filtered for use.

**Table 2. 0.4% Ketorolac Tromethamine Ophthalmic Solution (Comparative)**

| | |
|---|---|
| Ketorolac Tromethamine | 0.4% |
| Edetate Disodium | 0.015% |
| NaCl | 0.79% |
| Benzalkonium Chloride | 0.006% |
| Octoxynol-40 | 0.003% |
| Ph | 7.4 |

### Example 3

Example 3 does not form part of the invention and is provided for information purposes only. This example was prepared according to the procedure of Example 1, except that hydroxypropyl cellulose was used in place of the sodium carboxymethyl cellulose in an amount sufficient to provide a viscosity equivalent to the viscosity of the composition of Example 1.

### Example 4

The following composition was manufactured on a volume basis at ambient temperates from two principal parts. Each part is manufactured separately and then combined under controlled sequences to form a sterile bulk product: the first part (Part 1) involves the dissolution of carboxymethylcellulose sodium in water followed by bulk heat sterilization, and the second part (Part 2) involves dissolution of keterolac tromethamine and salts in water sterile filtration throng a 0.2 micron membrane into a sterile pressure vessel. The sterile bulk solution is then clarity filtered through a 20 micron polypropylene membrane filter into the filling machine reservoir.

The sterile post-clarity filtered solution is then filled by a UD filling machine via blow-fill-seal process into UD vials using virgin LDPE resin without colorant. The filling is done in an ISO Class 5 environment. The nominal fill is 0.4 mL into 0.9 mL capacity vials.

**Table 3: 0.45 % w/v Keterolac Tromethamine Ophthalmic Solution**

| **Ingredient** | **Function** | **Concentration (% w/v)** |
|---|---|---|
| Keterolac tromethamine | Active | 0.45% |
| Carboxymethycellulose Sodium (Med. Viscosity) | Thickening Agent | 0.325% |
| Carboxymethycellulose Sodium (High Viscosity) | Thickening Agent | 0.175% |
| NaCl | Tonicity Agent | 0.7% |
| Sodium Citrate Dihydrate | Buffer | 0.2% |
| Sodium Hydroxide (1N) | pH adjustment | Adjust to pH 6.8 |
| Hydrochloric Acid (1N) | pH adjustment | Adjust to pH 6.8 |
| Purified Water | Vehicle | Q.S. |

### Example 5

Comparison of Aqueous Humor Ketorolac Pharmacokinetics Following a Single Ocular Instillation of 0.45% Ketorolac Tromethamine Formulations with Varying pH to Acular LS® in New Zealand White Rabbits.

### Study Objectives:

1) To compare aqueous humor ketorolac pharmacokinetics following a single ocular instillation of 0.45% ketorolac tromethamine formulations with varying pH and Acular LS® to New Zealand White rabbits;
2) This Example was designed to determine whether decreasing the pH of the composition would increase the absorption of ketorolac into the eye; and,
3) In addition, one arm of this trial was designed to test the effect of decreasing viscosity of the composition from 22 cps to 11 cps.

The specifics of this study are as follows:
Rabbit Aqueous Humor Ketorolac Concentrations following Administration of Three 0.45% Ketorolac Tromethamine Formulations and Acular LS

**Table 4**

| | |
|---|---|
| • Treatment Groups | ▪ 0.45% Ketorolac Tromethamine 22 cps pH=7.4 |
| | ▪ 0.45% Ketorolac Tromethamine 22 cps pH=7.2 |
| | ▪ 0.45% Ketorolac Tromethamine 22 cps pH=7.0 |
| | ▪ 0.45% Ketorolac Tromethamine 11 cps pH=7.0 |
| | ▪ 0.45% Ketorolac Tromethamine 22 cps pH=6.8 |
| | ▪ Acular LS pH=7.4 |
| • Dosing Route: | Topical ocular |
| • Animal Gender: | NZW Rabbits/Female |
| • Dosing Regimen | Single dose, bilateral |
| • Timepoints: | 1, 2 and 4 hrs post-dose |
| • # Rabbits: | 3 rabbits/timepoint +1 rabbit blank |
| | Total = 39 rabbits |
| • Tissues/Matrices: | Aqueous Humor |
| • Bioanalysis: | LC-MS/MS |
| • Data analysis: | AUC₀₋ₜ, Cmax |

The results of the study are reported in Table 5, below.

**Table 5**

| **PK Parameters** | | | |
|---|---|---|---|
| Formulation | AUC₀₋₄± SE (ng·h/ml) | Cₘₐₓ± SD (ng/ml) | Relative %F* |
| 0.45% CMC 22 cps pH 7.4 w.o "outlier" | 627±51 | 265±71 | 135 |
| 045% CMC 22 cps pH 7.4 | 713±96 | 322±153 | 153 |
| 045% CMC 22 cps pH 7.2 | 620±50 | 240±84 | 133 |
| 045% CMC 22 cps pH 7.0 | 658±73 | 268±125 | 142 |
| 045% CMC 22 cps pH 6.8 | 939±163 | 389±258 | 202 |
| 045% CMC 11 cps pH 7.0 | 649±74 | 347±218 | 139 |
| Acular LS® | 465±65 | 211±106 | 100 |

### Summary of the results:

The sodium carboxymethyl cellulose-containing formulations perform better than Acular LS® with a relative bioavailability ranging from 133% (0.45% Keto 22 cps pH 7.2) to 202% (0.45% Keto 22 cps pH 6.8). However, there is not a clear pH effect-because the 0.45% Keto 22 cps pH 7.4 has a relative bioavailability of 153%, although one anomalous result maybe driving this observation. Nevertheless, the solution having a pH of 6.8 shows the best bioavailability.

### Example 6

Example 6 does not form part of the invention and is provided for information purposes only. A multicenter, randomized, double-masked, parallel-group study is carried out using the 0.4% ketorolac tromethamine formulations of Examples 1 and 3. The study subjects consisted of 157 patients (78-79/group) undergoing unilateral PRK surgery. The key inclusion criteria for the study is that each subject a) is a candidate for unilateral photorefractive keratectomy surgery (PRK) within 7 days after the initial visit, b) have best-corrected ETDRS visual acuity of 20/100 or better, and c) is capable of wearing a soft bandage contact lens. Key exclusion criteria are a history of refractive ocular surgery and sensitivity to study medication or its vehicle, Tylenol #3®, or Ocuflox®. The patient demographics are shown in Table 6. A total of 157 patients are enrolled with an age range of 20-66 years. There are no significant demographic differences between treatment groups.

**Table 6: Patient Demographics**

| | n | % |
|---|---|---|
| Gender | | |
| Female | 91 | 58 |
| Male | 66 | 42 |
| Age, mean ± SD | 39 ± 10 | |
| Race | | |
| Caucasian | 148 | 94 |
| Black | 5 | 3 |
| Hispanic | 2 | 1 |
| Asian | 1 | 1 |
| Other | 1 | 1 |

Each subject receives the Ocuflox® 5 min prior to study medication. The study subjects then receive ketorolac tromethamine 0.4% ophthalmic solution of Example 1 or Example 3, 1 drop QID for up to 4 days. Then all subjects are then instructed to take Tylenol #3® as needed for intolerable pain (escape medication). Patients use electronic diaries with date and time stamp to record the ocular pain they experience as one of the following: no pain, mild, moderate, severe, intolerable.

The pain intensity is less for the subjects who receive the solution of Example 1 during the first 12 hours post-PRK compared to those who receive the solution of Example 3. In particular, during the first 12 hours post-PRK, the group that receive the solution of Example 1 had fewer patients with severe or intolerable pain compared with the receive the solution of Example 3. In particular, the median pain intensity reported by the group which receive the solution of Example 1 was 1 grade less than with the group which receive the solution of Example 3 (moderate vs. severe on a 5-point scale of 0 = no pain to 4 = intolerable pain). Additionally, pain intensity is also less for the group which receive compared with the group which receive the solution of Example 3.

This clinical study shows that the solution of example 1 provides a greater degree of absorption of ketorolac as compared to the solution without sodium carboxymethylcellulose despite the fact that the solutions have the same concentration of ketorolac and are at the same viscosity.

In summary, the 0.4% ketorolac formulation is clinically effective in treating post PRK ocular pain. In patients treated with 0.4 ketorolac tromethamine-the patients treated with the solution comprising sodium carboxymethyl cellulose experienced significantly greater and faster pain relief, and used less escape medication compared to the patients treated with the solution comprising hydroxypropylcellulose.

### Example 7

Rabbit Ocular Pharmacokinetic Evaluation of Keterolac Tromethamine 0.45%

**NZW Rabbits/Female**

| | |
|---|---|
| Dosing Regimen: | Single ocular dose, bilateral |
| Timepoints: | 0.5, 1, 2, 4, 8, 10 and 24 hrs post-dose |
| Tissues/Matrices: | Aqueous Humor and Iris-ciliary body |
| Bioanalysis: | LC-MS/MS |
| Data Analysis: | Pharmacokinetic analyses and simulation |

### Conclusions:

1) Increase in relative bioavailability of keterolac as compared to Acular LS®;
2) Increased ketorolac concentrations are maintained longer post-dose; and
3) Together these data support a reduction in dosing frequency from 4X/day to 2X/day.

**Table 12: Safety and Tolerability Results**

| **Variable** | **Ketorolac 0.45%** | **ACULAR LS 0.40%** |
|---|---|---|
| **Ocular AEs-Irritation** | **10.0% (2/20)** | **15.4% (6/39)** |
| **Symptoms-Burning/stinging (≥ 1 grade increase)** | **10.0% (2/20)** | **12.8% (5/39)** |
| **Bulbar hyperemia (≥ trace)** | **10.0% (2/20)** | **23.1% (9/39)** |
| **Ocular comfort (≥ comfortable)** | **90-100%** | **84-100%** |

### Conclusions:

Acular 0.45% is safe and well-tolerated when given 5 times over a half-day and compares very favorably to ACULAR LS.

### Example 8

### Corneal Wound Healing

Studies have reported that the corneal toxicity of topical NSAIDS may result from an upregulation of matrix metalloproteinases (MMPs), endopeptidases involved in the remodeling of the corneal extracellular matrix. The addition of topical ketorolac, as well as several other topical NSAIDS, has been shown in animal models to stimulate the production of MMP-1 (collagenase), MMP-2 (gelatinase), and MMP-8 (collagenase) in both intact and epithelial debrided corneas. As topical NSAIDs are commonly used in the perioperative setting, it is critical to develop formulations that do not inhibit epithelial healing.

Ophthalmic bromfenac 0.09% (Xibrom®; ISTA Pharmaceuticals, Irvine, CA)⁶ and nepafenac 0.01% (Nevanac®; Alcon Laboratories, Inc., Fort Worth, TX)⁷ are other preserved topical NSAIDs. It was hypothesized that the addition of CMC and removal of BAK in the ketorolac 0.45% formulation would lead to improved corneal epithelial wound healing compared to prior ketorolac formulations and compared to bromfenac and nepafenac. This hypothesis was tested using a porcine-derived, corneal organ culture model.

A porcine corneal model of wound healing was developed which has previously been used to assess the role of growth factors in corneal reepithelialization. Given the changes in the formulation of ketorolac, the present study was designed to compare the impact on wound healing of the new formulation of ketorolac 0.45% to prior generations of ketorolac (0.4% and 0.5%) as well as to bromfenac 0.09% and nepafenac 0.1%.

**Ophthalmic Study Drug Formulations**

| **Active Ingredient Brand Name** | **Ketorolac 0.45%³ Acuvail** | **Ketorolac 0.5%⁴ Acular** | **Ketorolac 0.4%⁵ Acular LS** | **Bromfenac0.09%⁶ Xibrom** | **Nepafenac 0.1%⁷ Nevanac** |
|---|---|---|---|---|---|
| **Inactive ingredients** | CMC, Na citrate | EDTA, octoxynol | EDTA, octoxynol | EDTA, boric acid, Na borate, Na sulfite, povidone, polysorbate 80 | EDTA, mannitol, carbomer 947P, NaCl, tyloxapol |
| **Preservative** | No preservative | BAK (0.01%) | BAK (0.006%) | BAK (0.005%) | BAK (0.005%) |
| **pH** | 6.8 | 7.4 | 7.4 | 8.3 | 7.4 |

| | | | | | |
|---|---|---|---|---|---|
| CMC = carboxymethylcellulose. BAK = benzalkonium chloride. EDTA = ethylenediaminetetraacetic acid (sodium edetate). | | | | | |

In this Example, epithelial debrided porcine corneas were exposed to different formulations of the ketorolac molecule, namely ketorolac 0.45% (Acuvail®, Allergan, Inc), ketorolac 0.4% (Acular LS®; Allergan, Inc.) and ketorolac 0.5% (Acular®, Allergan, Inc). In brief, porcine eyes were obtained from a local abattoir, transported to the laboratory on ice in a moist chamber, and processed for corneal culture on the same day. An epithelial wound was made by demarcating an area on the central cornea with a 5-mm trephine and then removing the epithelium within the circle with a small scalpel, leaving an intact basement membrane. Corneal-scleral rims, with approximately 4 mm of the limbal conjunctiva present, were excised and rinsed in sterilized phosphate-buffered saline. The excised corneas were placed epithelial-side down into a sterile cup.

The endothelial corneal concavity was then filled with Minimum Essential Medium (MEM) containing 1% agarose and 1 mg/ml rat tail tendon collagen maintained at 42°C. This mixture was allowed to gel. The cornea, along with its supporting gel, was inverted (endothelial side down) and then transferred to a 35-mm dish. The culture medium (∼ 2 ml) was added dropwise to the surface of central cornea until the limbal conjunctiva was covered, leaving the epithelium exposed to the air. The corneas were then cultured in a humidified 5% CO₂ incubator at 37°C in MEM for 24 hours to ensure initial wound recovery. The basic steps were carried out as follows:
- 5 mm central epithelial wound made in freshly isolated porcine cornea with a trephine.
- After 24 hours, corneas were incubated for 10 minutes in either:
   - 50 µL Minimum Essential Medium (MEM) (negative control)
   - Triton X-100 (positive control)
   - NSAID (ketorolac 0.45%, ketorolac 0.4%, or ketorolac 0.5%)
- Corneas rinsed twice with 3 mL PBS, and 2 mL fresh MEM was added.
- Corneas allowed to heal for another 24 hours prior to staining with Richardson's staining solutions.
- Remaining wound area was photographed and the intensity of staining was quantified using Adobe® Photoshop® and expressed as area in pixels.

After 24 hours, a 50-µL volume of MEM, Triton X-100 (1%; Dow Chemical Company, Midland, MI), or study drugs (ketorolac 0.45%, ketorolac 0.4%, ketorolac 0.5%, nepafanc 0.01%, or bromfenac 0.09%) was applied to the epithelial wound surface for 10 minutes. MEM alone was used as a negative control, and Triton X-100 was used as a positive control. Corneas were rinsed twice with 3 mL of phosphate-buffered saline and incubated in 2 mL of fresh MEM for an additional 24 hours prior to staining. The remaining wound area was then stained with Richardson staining solution and photographed.²⁰ The staining area was quantified (in pixels) using Adobe Photoshop® (Adobe Systems, Inc., San Jose, CA) as previously described. A total of five corneas was used in each treatment group.

Data were presented as the mean of remaining wound area ± standard deviation (SD). One way analysis of variance (ANOVA) with a Newman-Keuls posttest was used for statistical comparison among groups. A P-value of < .05 was considered statistically significant.

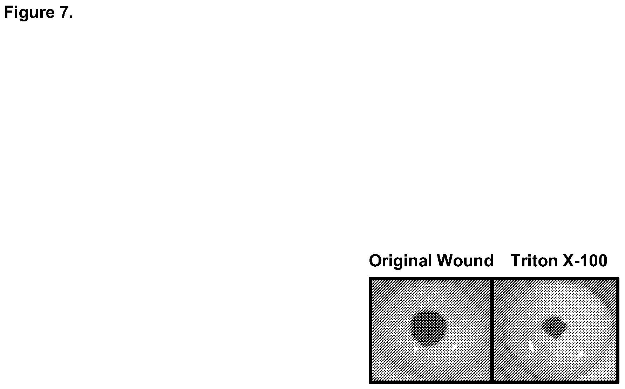

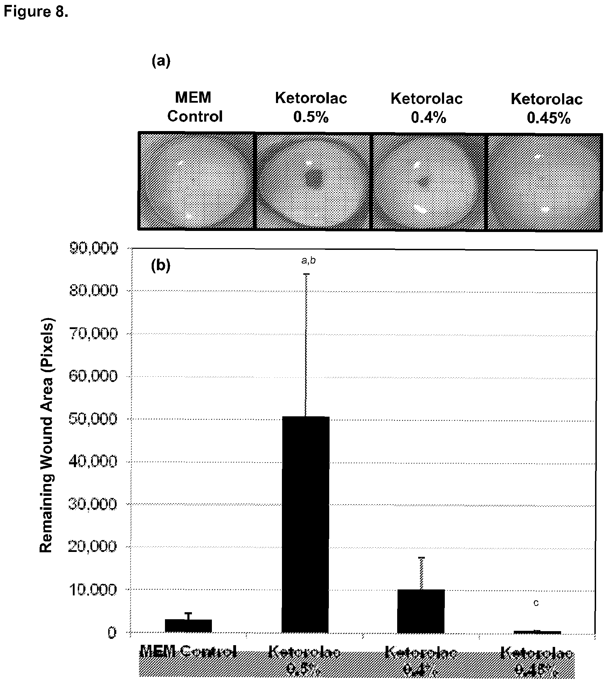

The mean (SD) original wound area induced by the 5 mm trephine was 200,506 ± 4,363 pixels. At 48 hours, corneas treated with Triton X-100 1% had a mean (SD) remaining wound area of 59,509 ± 4,850 pixels (Figure 7). The mean (SD) remaining wound areas at 48 hours following treatment with MEM control, ketorolac 0.45%, ketorolac 0.4%, and ketorolac 0.5% were 2,969 ± 1633 pixels, 586 ± 299 pixels, 10,228 ± 7,541 pixels, and 50,674 ± 33,409 pixels, respectively (Figure 8). Corneas treated with ketorolac 0.45% had a significantly smaller mean remaining wound area than did corneas treated with MEM control, ketorolac 0.4%, and ketorolac 0.5% (*P* < .05). In contrast, the mean remaining wound area of corneas treated with ketorolac 0.5% was not statistically different than those treated with Triton X-100 1%.

The results as shown in Figs. 7 and 8 demonstrate that ketorolac 0.45%-treated corneas demonstrated superior wound closure than the MEM negative control (no drug) (*P* < .05). Ketorolac 0.45%-treated corneas showed greater wound closure than the Triton X-100 positive control and prior ketorolac formulations (Acular®, Acular LS®) (P < .05).

Fig. 8 shows corneal wound area was significantly reduced in the ketorolac 0.45% group compared to eyes that received no drug (*P* < .05).
Wound area was also significantly reduced in the ketorolac 0.45% group compared to previous formulations (*P* < .05).

### Example 9

### Corneal Wound Healing

In this example, two parallel-group comparison studies were performed in series to evaluate epithelial wound closure in *ex vivo* porcine corneas treated with unpreserved ketorolac 0.45% compared with preserved ketorolac 0.4% and ketorolac 0.5% and ketorolac 0.45% compared with bromfenac 0.09% and nepafenac 0.1%. The basic steps were carried out as follows:
- A 5-mm central epithelial wound was made by demarcating an area on freshly isolated central porcine corneas with a trephine (n = 3-5 per group).
- Corneas were incubated in Minimum Essential Medium (MEM) in an air-lifted format for 24 hours to ensure initial wound recovery.
- A 50-µL volume of MEM, Triton™ X-100 detergent, or study drug was applied to the epithelial wound surface for 10 minutes. MEM alone was used as a negative control, and Triton X-100 was used as a positive control.
- Corneas were rinsed twice with 3 mL phosphate-buffered saline (PBS) and incubated in 2 mL of fresh MEM for an additional 24 hours prior to staining.
- The remaining wound area was then stained with Richardson staining solution and photographed.
- The intensity of the staining was quantified using Adobe Photoshop® (Adobe Systems, Inc.; San Jose, CA) and expressed as area in pixels.

The results demonstrate that ketorolac 0.45% leads to more rapid wound healing than prior formulations of ketorolac and both bromfenac and nepafenac in an animal model. Ketorolac 0.45%-treated corneas had similar or significantly greater wound closure than the MEM negative control (no drug). In this model, the enhanced wound closure in corneas exposed to ketorolac 0.45% compared to other agents may be attributed to the addition of carboxymethyl cellulose ("CMC") and the absence of BAK in its formulation. CMC is a commonly used viscosity-enhancing agent. In cell culture, CMC increases epithelial cell migration and cell proliferation.

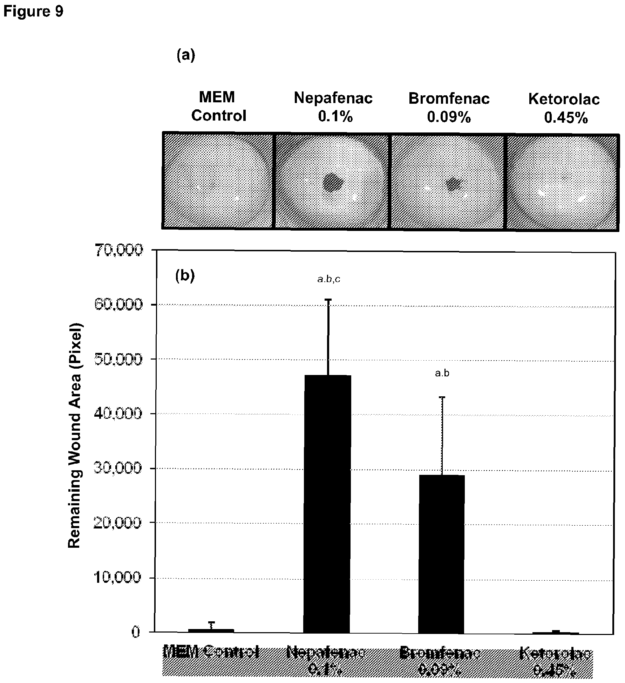

In Example 9, corneas treated with MEM control, ketorolac 0.45%, bromfenac 0.09%, and nepafenac 0.1% had mean (SD) remaining wound areas at 48 hours of 565 ± 1,263 pixels, 322 ± 229 pixels, 29,093 ± 14,295 pixels, and 47,322 ± 13,736 pixels, respectively (Figure 9). The remaining wound areas were significantly smaller in corneas treated with ketorolac 0.45% as compared to those treated with bromfenac 0.09% or nepafenac 0.1% (*P* < .01). Corneas treated with nepafenac 0.1 % had a significantly larger mean remaining wound area than those treated with bromfenac 0.09% (*P* < .01). The mean remaining wound area of corneas treated with nepafenac 0.1 % was not statistically different from that of corneas treated with Triton X-100 1%.

As shown in Figure 9, the mean remaining wound area was significantly reduced in corneas exposed to ketorolac 0.45% compared with corneas exposed to bromfenac, nepafenac, or Triton X-100 (*P* < .01). Compared to the MEM control (no drug), the remaining wound area in bromfenac-, nepafenac-, and Triton X-100-treated corneas was significantly greater (*P* < .001).The remaining wound area in corneas exposed to bromfenac compared with corneas exposed to nepafenac and Triton X-100 was significantly reduced (*P* < .01). No statistical difference was detected between the remaining wound areas of nepafenac- and Triton X-100-treated eyes.

### CONCLUSION

Compared with other NSAIDs and previous ketorolac formulations, ketorolac 0.45% provided more rapid epithelial wound healing in an animal model. Differences in wound healing may be due to the presence of CMC and/or absence of BAK in ketorolac 0.45%. The results demonstrate that ketorolac 0.45% led to more rapid wound healing than prior formulations of ketorolac in an animal model. The addition of CMC to the active agent led to more rapid wound healing than tissue culture medium alone. More rapid epithelial recovery was seen in ketorolac 0.45%- treated corneas than those treated with ketorolac 0.4%.

## Claims

1. A composition for use in a method of promoting corneal wound healing in a patient, wherein the composition comprises:
an aqueous solution of 0.45% w/v ketorolac tromethamine; and
carboxymethyl cellulose;
wherein the composition contains no preservative.

2. The composition for use according to Claim 1, wherein the composition comprises:
0.45% w/v ketorolac tromethamine;
0.325% w/v medium viscosity carboxymethyl cellulose sodium;
0.175% w/v high viscosity carboxymethyl cellulose sodium;
0.7% w/v NaCl; and
0.2% w/v sodium citrate dihydrate.

## Patentansprüche

1. Zusammensetzung zur Verwendung in einem Verfahren zur Förderung der Abheilung von Hornhautwunden bei einem Patienten, wobei die Zusammensetzung umfasst:
eine wässrige Lösung aus 0,45% m/V Ketorolac Tromethamin; und
Carboxymethylcellulose;
wobei die Zusammensetzung keinen Konservierungsstoff enthält.

2. Zusammensetzung zur Verwendung gemäß Anspruch 1, wobei die Zusammensetzung umfasst:
0,45% m/V Ketorolac Tromethamin;
0,325% m/V Natriumcarboxymethylcellulose einer mittleren Viskosität;
0,175% m/V Natriumcarboxymethylcellulose einer hohen Viskosität;
0,7% m/V NaCl; und
0,2% m/V Natriumcitrat-Dihydrat.

## Revendications

1. Composition pour une utilisation dans une méthode pour favoriser la cicatrisation de plaies cornéennes d'un patient, dans laquelle la composition comprend :
une solution aqueuse de 0,45 % p/v de kétorolac de trométhamine ; et
de la carboxyméthylcellulose;
dans laquelle la composition ne contient aucun conservateur.

2. Composition pour une utilisation selon la revendication 1, dans laquelle la composition comprend :
0,45 % p/v de kétorolac de trométhamine ;
0,325 % p/v de carboxyméthylcellulose sodique moyenne viscosité ;
0,175 % p/v de carboxyméthylcellulose sodique haute viscosité;
0,7 % p/v de NaCl ; et
0,2 % p/v de citrate de sodium dihydraté.
